# EUROPEAN PATENT SPECIFICATION

(11) **EP 1 974 210 B1**
(45) Date of publication and mention of the grant of the patent: **29.12.2010**
(21) Application number: 07700227.7
(22) Date of filing: 17.01.2007
(51) Int. Cl.: G01N 33/50, G01N 33/68

(54) **METHOD FOR DIAGNOSING POLYCYSTIC KIDNEY DISEASE**
VERFAHREN ZUR DIAGNOSE VON POLYZYSTISCHER NIERENKRANKHEIT
PROCEDE DE DIAGNOSTIC DE LA POLYKYSTOSE RENALE

(30) Priority: 17.01.2006 EP 06000963
(43) Date of publication of application: 01.10.2008
(73) Proprietor: Universitätsklinikum Freiburg, 79106 Freiburg (DE)
(72) Inventor: WALZ, Gerd, 79104 Freiburg (DE)
(74) Representative: Kalhammer, Georg
(86) International application number: PCT/EP2007/000384
(87) International publication number: WO 2007/082733

(56) References cited:
- EP-A- 0 879 885
- WO-A-02/05857
- WO-A-2004/011618
- WO-A-2005/070448
- WO-A-2005/117941
- US-A1- 2003 143 610
- US-A1- 2004 191 818
- US-B1- 6 638 726
- BERNDT THERESA ET AL: "Secreted frizzled-related protein 4 is a potent tumor-derived phosphaturic agent." THE JOURNAL OF CLINICAL INVESTIGATION. SEP 2003, vol. 112, no. 5, September 2003 (2003-09), pages 785-794, XP002371869 ISSN: 0021-9738 cited in the application
- DONAUER J ET AL: "EXPRESSION PROFILING OF POLCYSTIC KIDNEY DISEASE" JOURNAL OF THE AMERICAN SOCIETY OF NEPHROLOGY, WILLIAMS AND WILKINS, BALTIMORE, MD, US, vol. 14, no. ISSUE, November 2003 (2003-11), page 106A, XP008061318 ISSN: 1046-6673
- WILSON P D: "EPITHELIAL CELL POLARITY AND DISEASE" AMERICAN JOURNAL OF PHYSIOLOGY: RENAL, FLUID AND ELECTROLYTE PHYSIOLOGY, AMERICAN PHYSIOLOGICAL SOCIETY, US, vol. 272, no. 4, PART 2, 1997, pages F434-F442, XP001021144 ISSN: 0363-6127
- ZHANG QIHONG ET AL: "Cystic kidney diseases: all roads lead to the cilium." PHYSIOLOGY (BETHESDA, MD.) AUG 2004 LNKD- PUBMED:15304637, vol. 19, August 2004 (2004-08), pages 225-230, ISSN: 1548-9213

### SEQUENCE LISTING

<110> Universitaetsklinikum Freiburg
<120> Method for Diagnosing Polycystic Kidney Disease
<130> Prof. Dr. walz
<160> 14
<170> PatentIn version 3.3
<210> 1
   <211> 314
   <212> PRT
   <213> Homo sapiens
<400> 1
   Met Gly Ile Gly Arg Ser Glu Gly Gly Arg Arg Gly Ala Ala Leu Gly
   1               5                   10                  15
   Val Leu Leu Ala Leu Gly Ala Ala Leu Leu Ala Val Gly Ser Ala Ser
               20                  25                  30
   Glu Tyr Asp Tyr Val Ser Phe Gln Ser Asp Ile Gly Pro Tyr Gln Ser
           35                  40                  45
   Gly Arg Phe Tyr Thr Lys Pro Pro Gln Cys Val Asp Ile Pro Ala Asp
       50                  55                  60
   Leu Arg Leu Cys His Asn Val Gly Tyr Lys Lys Met Val Leu Pro Asn
   65                  70                  75                  80
   Leu Leu Glu His Glu Thr Met Ala Glu Val Lys Gln Gln Ala Ser Ser
                   85                  90                  95
   Trp Val Pro Leu Leu Asn Lys Asn Cys His Ala Gly Thr Gln Val Phe
               100                 105                 110
   Leu Cys Ser Leu Phe Ala Pro Val Cys Leu Asp Arg Pro Ile Tyr Pro
           115                 120                 125
   Cys Arg Trp Leu Cys Glu Ala Val Arg Asp Ser Cys Glu Pro Val Met
       130                 135                 140
   Gln Phe Phe Gly Phe Tyr Trp Pro Glu Met Leu Lys Cys Asp Lys Phe
   145                 150                 155                 160
   Pro Glu Gly Asp Val Cys Ile Ala Met Thr Pro Pro Asn Ala Thr Glu
                   165                 170                 175
   Ala Ser Lys Pro Gln Gly Thr Thr Val Cys Pro Pro Cys Asp Asn Glu
               180                 185                 190
   Leu Lys Ser Glu Ala Ile Ile Glu His Leu Cys Ala Ser Glu Phe Ala
           195                 200                 205
   Leu Arg Met Lys Ile Lys Glu Val Lys Lys Glu Asn Gly Asp Lys Lys
       210                 215                 220
   Ile Val Pro Lys Lys Lys Lys Pro Leu Lys Leu Gly Pro Ile Lys Lys
   225                 230                 235                 240
   Lys Asp Leu Lys Lys Leu Val Leu Tyr Leu Lys Asn Gly Ala Asp Cys
                   245                 250                 255
   Pro Cys His Gln Leu Asp Asn Leu Ser His His Phe Leu Ile Met Gly
               260                 265                 270
   Arg Lys Val Lys Ser Gln Tyr Leu Leu Thr Ala Ile His Lys Trp Asp
           275                 280                 285
   Lys Lys Asn Lys Glu Phe Lys Asn Phe Met Lys Lys Met Lys Asn His
       290                 295                 300
   Glu Cys Pro Thr Phe Gln Ser Val Phe Lys
   305                 310
<210> 2
   <211> 295
   <212> PRT
   <213> Homo sapiens
<400> 2
<210> 3
   <211> 325
   <212> PRT
   <213> Homo sapiens
<400> 3
   Met Val Cys Gly Ser Pro Gly Gly Met Leu Leu Leu Arg Ala Gly Leu
   1               5                   10                  15
   Leu Ala Leu Ala Ala Leu Cys Leu Leu Arg Val Pro Gly Ala Arg Ala
               20                  25                  30
   Ala Ala Cys Glu Pro Val Arg Ile Pro Leu Cys Lys Ser Leu Pro Trp
           35                  40                  45
   Asn Met Thr Lys Met Pro Asn His Leu His His Ser Thr Gln Ala Asn
       50                  55                  60
   Ala Ile Leu Ala Ile Glu Gln Phe Glu Gly Leu Leu Gly Thr His Cys
   65                  70                  75                  80
   Ser Pro Asp Leu Leu Phe Phe Leu Cys Ala Met Tyr Ala Pro Ile Cys
                   85                  90                  95
   Thr Ile Asp Phe Gln His Glu Pro Ile Lys Pro Cys Lys Ser Val Cys
               100                 105                 110
   Glu Arg Ala Arg Gln Gly Cys Glu Pro Ile Leu Ile Lys Tyr Arg His
           115                 120                 125
   Ser Trp Pro Glu Asn Leu Ala Cys Glu Glu Leu Pro Val Tyr Asp Arg
       130                 135                 140
   Gly Val Cys Ile Ser Pro Glu Ala Ile Val Thr Ala Asp Gly Ala Asp
   145                 150                 155                 160
   Phe Pro Met Asp Ser Ser Asn Gly Asn Cys Arg Gly Ala Ser Ser Glu
                   165                 170                 175
   Arg Cys Lys Cys Lys Pro Ile Arg Ala Thr Gln Lys Thr Tyr Phe Arg
               180                 185                 190
   Asn Asn Tyr Asn Tyr Val Ile Arg Ala Lys Val Lys Glu Ile Lys Thr
           195                 200                 205
   Lys Cys His Asp Val Thr Ala Val Val Glu Val Lys Glu Ile Leu Lys
       210                 215                 220
   Ser Ser Leu Val Asn Ile Pro Arg Asp Thr Val Asn Leu Tyr Thr Ser
   225                 230                 235                 240
   Ser Gly Cys Leu Cys Pro Pro Leu Asn Val Asn Glu Glu Tyr Ile Ile
                   245                 250                 255
   Met Gly Tyr Glu Asp Glu Glu Arg Ser Arg Leu Leu Leu Val Glu Gly
               260                 265                 270
   Ser Ile Ala Glu Lys Trp Lys Asp Arg Leu Gly Lys Lys Val Lys Arg
           275                 280                 285
   Trp Asp Met Lys Leu Arg His Leu Gly Leu Ser Lys Ser Asp Ser Ser
       290                 295                 300
   Asn Ser Asp Ser Thr Gln Ser Gln Lys Ser Gly Arg Asn Ser Asn Pro
   305                 310                 315                 320
   Arg Gln Ala Arg Asn
                   325
<210> 4
   <211> 346
   <212> PRT
   <213> Homo sapiens
<400> 4
   Met Phe Leu Ser Ile Leu Val Ala Leu Cys Leu Trp Leu His Leu Ala
   1               5                   10                  15
   Leu Gly Val Arg Gly Ala Pro Cys Glu Ala Val Arg Ile Pro Met Cys
               20                  25                  30
   Arg His Met Pro Trp Asn Ile Thr Arg Met Pro Asn His Leu His His
           35                  40                  45
   Ser Thr Gln Glu Asn Ala Ile Leu Ala Ile Glu Gln Tyr Glu Glu Leu
       50                  55                  60
   Val Asp Val Asn Cys Ser Ala Val Leu Arg Phe Phe Leu Cys Ala Met
   65                  70                  75                  80
   Tyr Ala Pro Ile Cys Thr Leu Glu Phe Leu His Asp Pro Ile Lys Pro
                   85                  90                  95
   Cys Lys Ser Val Cys Gln Arg Ala Arg Asp Asp Cys Glu Pro Leu Met
               100                 105                 110
   Lys Met Tyr Asn His Ser Trp Pro Glu Ser Leu Ala Cys Asp Glu Leu
           115                 120                 125
   Pro Val Tyr Asp Arg Gly Val Cys Ile Ser Pro Glu Val Ile Val Thr
       130                 135                 140
   Asp Leu Pro Glu Asp Val Lys Trp Ile Asp Ile Thr Pro Asp Met Met
   145                 150                 155                 160
   Val Gln Glu Arg Pro Leu Asp Val Asp Cys Lys Arg Leu Ser Pro Asp
                   165                 170                 175
   Arg Cys Lys Cys Lys Lys Val Lys Pro Thr Leu Ala Thr Tyr Leu Ser
               180                 185                 190
   Lys Asn Tyr Ser Tyr Val Ile His Ala Lys Ile Lys Ala Val Gln Arg
           195                 200                 205
   Ser Gly Cys Asn Glu Val Thr Thr Val Val Asp Val Lys Glu Ile Phe
       210                 215                 220
   Lys Ser Ser Ser Pro Ile Pro Arg Thr Gln Val Pro Leu Ile Thr Asn
   225                 230                 235                 240
   Ser Ser Cys Gln Cys Pro His Ile Leu Pro His Gln Asp Val Leu Ile
                   245                 250                 255
   Met Cys Tyr Glu Trp Arg Ser Arg Met Met Leu Leu Glu Asn Cys Leu
   260 265 270
   Val Glu Lys Trp Arg Asp Gln Leu Ser Lys Arg Ser Ile Gln Trp Glu
   275 280 285
   Glu Arg Leu Gln Glu Gln Arg Arg Thr Val Gln Asp Lys Lys Lys Thr
   290 295 300
   Ala Gly Arg Thr Ser Arg Ser Asn Pro Pro Lys Pro Lys Gly Lys Pro
   305 310 315 320
   Pro Ala Pro Lys Pro Ala Ser Pro Lys Lys Asn Ile Lys Thr Arg Ser
   325 330 335
   Ala Gln Lys Arg Thr Asn Pro Lys Arg Val
   340 345
<210> 5
   <211> 317
   <212> PRT
   <213> Homo sapiens
<400> 5
   Met Arg Ala Ala Ala Ala Ala Gly Gly Val Arg Thr Ala Ala Leu Ala
   1 5 10 15
   Leu Leu Leu Gly Ala Leu His Trp Ala Pro Ala Arg Cys Glu Glu Tyr
   20 25 30
   Asp Tyr Tyr Gly Trp Gln Ala Glu Pro Leu His Gly Arg Ser Tyr Ser
   35 40 45
   Lys Pro Pro Gln Cys Leu Asp Ile Pro Ala Asp Leu Pro Leu Cys His
   50 55 60
   Thr Val Gly Tyr Lys Arg Met Arg Leu Pro Asn Leu Leu Glu His Glu
   65 70 75 80
   Ser Leu Ala Glu Val Lys Gln Gln Ala Ser Ser Trp Leu Pro Leu Leu
   85 90 95
   Ala Lys Arg Cys His Ser Asp Thr Gln Val Phe Leu Cys Ser Leu Phe
   100 105 110
   Ala Pro Val Cys Leu Asp Arg Pro Ile Tyr Pro Cys Arg Ser Leu Cys
   115 120 125
   Glu Ala Val Arg Ala Gly Cys Ala Pro Leu Met Glu Ala Tyr Gly Phe
   130 135 140
   Pro Trp Pro Glu Met Leu His Cys His Lys Phe Pro Leu Asp Asn Asp
   145 150 155 160
   Leu Cys Ile Ala Val Gln Phe Gly His Leu Pro Ala Thr Ala Pro Pro
   165 170 175
   Val Thr Lys Ile Cys Ala Gln Cys Glu Met Glu His Ser Ala Asp Gly
   180 185 190
   Leu Met Glu Gln Met Cys Ser Ser Asp Phe Val Val Lys Met Arg Ile
   195 200 205
   Lys Glu Ile Lys Ile Glu Asn Gly Asp Arg Lys Leu Ile Gly Ala Gln
   210 215 220
   Lys Lys Lys Lys Leu Leu Lys Pro Gly Pro Leu Lys Arg Lys Asp Thr
   225 230 235 240
   Lys Arg Leu Val Leu His Met Lys Asn Gly Ala Gly Cys Pro Cys Pro
   245 250 255
   Gln Leu Asp Ser Leu Ala Gly Ser Phe Leu Val Met Gly Arg Lys Val
   260 265 270
   Asp Gly Gln Leu Leu Leu Met Ala Val Tyr Arg Trp Asp Lys Lys Asn
   275 280 285
   Lys Glu Met Lys Phe Ala Val Lys Phe Met Phe Ser Tyr Pro Cys Ser
   290 295 300
   Leu Tyr Tyr Pro Phe Phe Tyr Gly Ala Ala Glu Pro His
   305 310 315
<210> 6
   <211> 20
   <212> PRT
   <213> Artificial
<220>
   <223> C-terminal peptide of sFRP4
<400> 6
   Arg Thr Ser Arg Ser Asn Pro Pro Lys Pro Lys Gly Lys Pro Pro Ala
   1 5 10 15
   Pro Lys Pro Ala
   20
<210> 7
   <211> 16
   <212> PRT
   <213> Artificial
<220>
   <223> C-terminal peptide of sFRP4
<400> 7
   Pro Lys Lys Asn Ile Lys Thr Arg Ser Ala Arg Lys Arg Thr Asn Pro
   1 5 10 15
<210> 8
   <211> 20
   <212> DNA
   <213> artificial
<220>
   <223> Primer 1 for sFRP4 amplification
<400> 8
   atcggtgcaa gtgcaaaaag 20
<210> 9
   <211> 20
   <212> DNA
   <213> Artificial
<220>
   <223> Primer 2 for sFRP4 amplification
<400> 9
   tatgtggaca ctggcaggag 20
<210> 10
   <211> 21
   <212> DNA
   <213> Artificial
<220>
   <223> Primer 1 for GASP2 amplification
<400> 10
   cttcaggtgt acatgaataa a 21
<210> 11
   <211> 24
   <212> DNA
   <213> Artificial
<220>
   <223> Primer 2 for GASP2 amplification
<400> 11
   gaggtcgtgg tgatttgcta aggc 24
<210> 12
   <211> 23
   <212> DNA
   <213> Artificial
<220>
   <223> Primer 1 for GASP9 amplification
<400> 12
   gcctctgcta atggcggaca ggc 23
<210> 13
   <211> 21
   <212> DNA
   <213> Artificial
<220>
   <223> Primer 2 for GASP9 amplification
<400> 13
   gggaggcctc ccaattggcg g 21
<210> 14
   <211> 15
   <212> PRT
   <213> Artificial sequence
<220>
   <223> N-terminal peptide of sFRP4
<400> 14
   Pro Leu Met Lys Met Tyr Asn His Ser Trp Pro Glu Ser Leu Ala
   1               5                   10                  15

## Description

The present invention relates to the diagnosis of polycystic kidney diseases (PKD). The invention provides methods for diagnosing or monitoring the progression of PKD and the use of test kits in those methods.

PKD is a common hereditary disease associated with renal failure. Although cyst growth and compression of surrounding tissue may account for some loss of renal tissue, the nature of the progressive renal failure in patients with PKD is unknown.

Autosomal dominant polycystic kidney disease (ADPKD) occurs in approximately 1 of 1000 humans, and causes end-stage renal disease (ESRD) in more than 50% of all affected patients. Cyst formation initiates during embryogenesis, but typically does not compromise renal function until later in life. Mutations of either *PKD1* or *PKD2* cause the disease, but why cysts present in less than one percent of all nephrons cause renal failure remains elusive. Although persistent proliferation, fluid secretion and cyst expansion may damage the surrounding tissue by reactive changes of the extracellular matrix, increased apoptosis of healthy parenchyma appears to be the main culprit of progressive renal failure. Epithelial cells isolated from cystic kidneys show increased levels of proto-oncogene expression, a mis-localization of integral membrane proteins and active fluid secretion, but these alterations do not explain the accelerated tissue loss *in vivo.*

Medicaments are being developed for which a therapeutic effect in the treatment of PKD can be expected (e.g., vasopressin-2 receptor antagonists and mTOR inhibitors). Clinical phase II/III studies for these substances will be launched next year. It is not yet known, however, how the short term therapeutic efficiency could be monitored (i.e. within a period of about one to about four years). As the disease develops over many years, it is difficult to determine whether or not a therapeutic intervention efficiently inhibits the progression of the disease. Currently, the measurement of the amount of creatinine in serum (creatinine clearance) as a marker of renal function is the only method available. This method, however, has the disadvantage that renal function remains normal although the cysts have already developed dramatically over years. As a consequence, measurement of creatinine in serum is not suitable for monitoring the progression of PKD or for determining the therapeutic efficiency of a medicament.

It is therefore one object of the present invention to provide a method for determining the efficiency of a therapeutic treatment of PKD. Another object of this invention is to provide a method for diagnosing PKD.

It has surprisingly been found that cyst formation is associated with increased production and accumulation of secreted frizzled related protein-4 (sFRP4). sFRP4 is a member of an evolving family of soluble molecules that bind both Wnt and frizzled molecules. sFRP4 is a soluble factor that antagonizes Wnt signaling and causes apoptosis. In addition, it has unexpectedly been found that sFRP4 and other members of the family of secreted frizzled related proteins are present in the serum and excreted in the urine of patients with PKD and that the sFRP4 production correlates with the extent of cyst volume and stage of renal failure. Furthermore, sFRP5 can be specifically detected in the urine of PKD patients.

The present invention therefore relates to a method for diagnosing or monitoring the progression of PKD as defined in the claims. The PKD may be acquired or inherited. The PKD may be associated with or caused by mutations in the gene(s) *PKD1* and/or *PKD2.* Accordingly, in a preferred embodiment the present invention relates to a method for diagnosing or monitoring the progression of PKD, comprising detecting secreted frizzled related protein 4 having the amino acid sequence as shown in SEQ ID NO: 4 in a sample containing wire or serum obtained from an individual suffering from PKD or suspected of suffering from PKD.

Donauer et al. (2003), Journal of the American Society of Nephrology, vol. 14, Abstracts issue, page 106A describe that sFRP4 mRNA is upregulated in kidneys removed from patients with polycystic kidney disease (PKD). However, only mRNA was detected, but not protein. In many cases, verification of microarray data has revealed that the mRNA of upregulated genes does not necessarily translate into increased production of protein. Second, the kidneys examined in that paper were removed from patients with end-stage renal disease (ESRD). The findings on this population are not necessarily reflective of the changes in gene expression and production of functioning ADPKD kidneys. Third, the detection of sFRP4 by RT-PCR does not involve antibody-antigen complexes, but is entirely based on reverse transcription of mRNA into cDNA, and detection of this cDNA by PCR and gel electrophoresis. Immunochemistry was not performed for sFRP4. The present invention does not relate to the discovery that mRNA is upregulated in PKD patients with end-stage renal disease, but is based on increased production of sFRP4 in PKD patients with functioning kidneys. Numerous genes are likely to be induced by uremia and/or renal replacement therapy, and specificity remains questionable. The finding that sFRP4 as well as other members of the sFRP family are detectable in the urine and the blood of patients with functioning kidneys has not been reported in the prior art.

Berndt et al. (2003) The Journal of Clinical Investigation 112(5), 785 describe studies on the phospaturic activity of sFRP4. These studies, however, were not related to PKD. Furthermore, this document does not disclose that sFRP4 may be present in the serum or urine of subjects suffering from a cystic kidney disease.

Zheng et al. (J.Am.Soc.Nephrol. 14: 2588-2595, 2003) describe studies on urinary excretion of monocyte chemoattractant protein-1 (MCP-1) in ADPKD. This publication does not disclose the detection of sFRP4 or sFRP5.

WO 02/05857 A2 discloses the human FRP-4 amino acid sequence and describes a possible role of this protein in phosphate homeostasis and transport. It does not teach detecting sFRP4 or sFRP5 for monitoring or diagnosing PKD.

The terms "secreted frizzled related protein" and "sFRP" are used interchangeably herein. sFRPs include, but are not limited to, sFRP1, sFRP2, sFRP3, sFRP4, and sFRP5. The sFRP is preferably capable of binding both Wnt and one or more frizzled molecules.

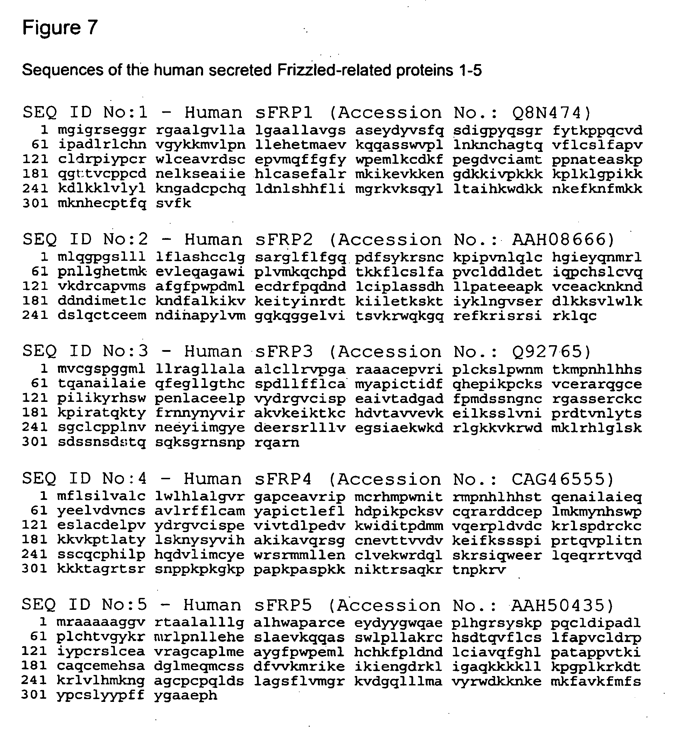

The term "secreted frizzled related protein-1" or "sFRP1" denotes a protein having substantially the amino acid sequence as shown in SEQ ID NO:1 (Figure 7). Preferably, sFRP1 is a protein substantially consisting of the amino acid sequence as shown in SEQ ID NO:1. sFRP1 also includes protein variants of the sequence as shown in SEQ ID NO:1, for example due to allelic variation. The variants may have mutations relative to the sequence as shown in SEQ ID NO:1, e.g. substitutions, additions and/or deletions. One to 30, or one to 20, or one to ten, or one to five, or one to three amino acids may be substituted, deleted and/or added relative to the sequence as shown in SEQ ID NO:1.

The term "secreted frizzled related protein-2" or "sFRP2" denotes a protein having substantially the amino acid sequence as shown in SEQ ID NO:2 (Figure 7). Preferably, sFRP2 is a protein substantially consisting of the amino acid sequence as shown in SEQ ID NO:2. sFRP2 also includes protein variants of the sequence as shown in SEQ ID NO:2, for example due to allelic variation. The variants may have mutations relative to the sequence as shown in SEQ ID NO:2, e.g. substitutions, additions and/or deletions. One to 30, or one to 20, or one to ten, or one to five, or one to three amino acids may be substituted, deleted and/or added relative to the sequence as shown in SEQ ID NO:2.

The term "secreted frizzled related protein-3" or "sFRP3" denotes a protein having substantially the amino acid sequence as shown in SEQ ID NO:3 (Figure 7). Preferably, sFRP3 is a protein substantially consisting of the amino acid sequence as shown in SEQ ID NO:3. sFRP3 also includes protein variants of the sequence as shown in SEQ ID NO:3, for example due to allelic variation. The variants may have mutations relative to the sequence as shown in SEQ ID NO:3, e.g. substitutions, additions and/or deletions. One to 30, or one to 20, or one to ten, or one to five, or one to three amino acids may be substituted, deleted and/or added relative to the sequence as shown in SEQ ID NO:3.

The term "secreted frizzled related protein-4" or "sFRP4" denotes a protein having the amino acid sequence as shown in SEQ ID NO:4 (Figure 7). Preferably, sFRP4 is a protein consisting of the amino acid sequence as shown in SEQ ID NO:4.

The term "secreted frizzled related protein-5" or "sFRP5" denotes a protein having substantially the amino acid sequence as shown in SEQ ID NO:5 (Figure 7). Preferably, sFRP5 is a protein substantially consisting of the amino acid sequence as shown in SEQ ID NO:5. sFRP5 also includes protein variants of the sequence as shown in SEQ ID NO:5, for example due to allelic variation. The variants may have mutations relative to the sequence as shown in SEQ ID NO:5, e.g. substitutions, additions and/or deletions. One to 30, or one to 20, or one to ten, or one to five, or one to three amino acids may be substituted, deleted and/or added relative to the sequence as shown in SEQ ID NO:5.

The method disclosed may comprise detecting one sFRP in the sample. Alternatively, the method may comprise detecting at least two different sFRPs in the sample. The method may comprise detecting at least three, at least four or at least five different sFRPs in the sample. The method comprises detecting human sFRP4 in the sample. In another preferred embodiment according to dependent claim 8, the method comprises detecting sFRP4 and sFRP5 in the sample.

The term "sample" as used herein designates a composition which is derived from the body of an individual. Usually, the individual has functioning kidneys. In a specific embodiment, the individual does not have end-stage renal disease (ESRD). Preferred samples are urine samples and serum samples from the individual. The sample may be a composition which has been processed to be in a condition suitable for the method according to the invention. For example, a blood sample may be subject to centrifugation such that serum is obtained, or a urine sample may be diluted prior to analysis (e.g. by 1:2 to 1:100, or by 1:5 or 1:50 or by 1:10 to 1:20). The processing may include centrifugation, precipitation, concentration, filtration, dialysis and/or dilution. The type of processing may depend on the technique which is used for detecting the sFRP in the sample.

The method of the invention comprises only steps which are carried out *in vitro.* The step of obtaining the sample from the body of the individual is not encompassed by the present invention.

The step of detecting sFRP4 in the sample may include determining the presence or absence of sFRP4 in the sample in a qualitative manner. The step of detecting sFRP4, however, may also include determining the amount or concentration of sFRP4 in the sample in a quantitative or semiquantitative manner. The step of detecting sFRP4 includes detecting fragments of sFRP4.

The amount of sFRP4 in the urine of a healthy individual is not detectable by Western blot analysis. The amount of sFRP4 in the urine of a patient suffering from PKD ranges from < 10 ng/ml to > 50 µg/ml, for example from about 10 ng/ml to about 50 µg/ml. Thus, a patient with PKD may excrete between < 1 µg and > 50 mg per day, for example from about 1 µg to about 50 mg per day. Since the urine concentration varies, the amount of sFRP4 excreted per day may correlate more precisely with the stage of the disease. In renal cystic disease, most cysts become disconnected from the draining tubular system. Thus, measurements of sFRP4 in the serum rather than in the urine may provide a more accurate estimate of cyst volumen and disease progression. In the serum, the sFRP4 concentration can range from < 10 ng/ml to > 100 ng/ml, e.g. from about 10 ng/ml to about 100 ng/ml, in patients suffering from PKD, and is not detectable in patients without kidney disease.

In one aspect of the invention the detection of an elevated level of sFRP4 according to dependent claim 4, in the sample from the individual relative to control samples indicates a diagnosis of PKD. Likewise, the extent of elevation of sFRP4, in the sample from the individual relative to control samples may correlate to the progression of PKD in the individual. The control samples have been obtained from individuals not suffering from renal diseases such as PKD. Concentrations of sFRP4 in the sample > 50 ng/ml are typically associated with renal cysts independent of the etiology.

The concentration ranges mentioned hereinabove with respect to sFRP4 may be applied to sFRP5 as well.

The method of the invention preferably comprises at least one direct or indirect immunoassay. These assays include but are not limited to competitive binding assays, non-competitive binding assays, radioimmunoassays, enzyme-linked immunosorbent assays (ELISA), sandwich assays, precipitin reactions, gel diffusion immunodiffusion assays, agglutination assays, fluorescence immunoassays, chemoluminescence immunoassays, immunoPCR immunoassays, protein A or protein G immunoassays and immunoelectrophoresis assays. Among all, enzyme immunoassay, in particular, enzyme-linked immunosorbent assay (ELISA) is appropriately used in hospitals, etc., since sFRP4 can be detected with high sensitivity and a number of specimens can be automatically assayed thereby.

The immunoassay comprises the use of a polyclonal or monoclonal antibody capable of binding to human sFRP4. As used herein, the term "antibody" designates an immunoglobulin or a derivative thereof having the same binding specificity. The antibody used in the invention may be a monoclonal antibody or an antibody derived from or comprised in a polyclonal antiserum, monoclonal antibodies are preferred. The term "antibody", as used herein, further comprises derivatives such as Fab, F(ab')₂, Fv or scFv fragments: see, for example Harlow and Lane, "Antibodies, A Laboratory Manual" CSH Press 1988, Cold Spring Harbor N.Y. Fab, F(ab')₂ and Fv fragments fragments can be obtained by digesting a complete antibody with papain, pepsin, etc. in the conventional manner. The antibody or the derivative thereof may be of natural origin or may be (semi)synthetically produced. Such synthetic products also comprises non-proteinaceous or semi-proteinaceous material that has the same or essentially the same binding specificity as the antibody used in the invention. Such products may, for example be obtained by peptidomimetics.

The antibody may be capable of binding to one or more of sFRP1, sFRP2, sFRP3, sFRP4 and sFRP5.

The antibody against sFRP4 to be used in the present invention can be produced by immunizing an animal such as rabbit, rat, goat, sheep or mouse with sFRP4 or a fragment thereof or a derivative thereof by a method well known by those skilled in the art (Harlow and Lane, "Antibodies, A Laboratory Manual" CSH Press 1988, Cold Spring Harbor N.Y.). The antigen to be used for immunization is preferably human sFRP4 or a fragment thereof or a derivative thereof. sFRP4 from other species, however, may also be used. The antigen may be prepared by recombinant expression in a host, e.g. in bacteria, insect cells, or mammalian cells, or it may be prepared by chemical synthesis by the solid phase method. Techniques for producing monoclonal antibodies are known to those skilled in the art.

The antibody to be used in accordance with this disclosure may recognize only one of sFRP1, sFRP2, sFRP3, sFRP4 and sFRP5. For example, the antibody may be capable of binding to sFRP4 but does not bind to sFRP1, sFRP2, sFRP3 or sFRP5. Alternatively, the antibody may recognize only two, only three, or only four of sFRP1, sFRP2, sFRP3, sFRP4 and sFRP5. In yet another embodiment, the antibody is a pan-reactive antibody which recognizes each of sFRP1, sFRP2, sFRP3, sFRP4 and sFRP5. The present disclosure relates to such antibodies described above.

The antibody to be used in accordance with this invention may be specific to human sFRP4, i.e. it does not recognize other proteins in the sample. Most preferably, the antibody does not recognize other proteins in human urine. The sFRP4-specific antibody may bind to an epitope in one of the following regions of the sFRP4 molecule: carboxy-terminal peptide of sFRP4 - (amino acids 307 - 326): RTSRSNPPKPKGKPPAPKPA (SEQ ID NO:6), (amino acids 328 - 343): PKKNIKTRSARKRTNP (SEQ ID NO:7), and amino terminal peptide of sFRP4 - (amino acids 110-124): PLMKMYNHSWPESLA (SEQ ID NO: 14).

In the ELISA method, an antibody, preferably a monoclonal antibody, or a fragment thereof against sFRP is usually first immobilized on a carrier as a primary antibody. Preferred carrier is a solid carrier, such as an ELISA plate container molded from a carrier polymer such as styrene or polystyrene. The monoclonal antibody or its fragment can be immobilized by, for example, dissolving the monoclonal antibody or its fragment in a buffer such as a carbonate buffer or a borate buffer followed by the adsorption onto the carrier.

Separately, an antibody (a monoclonal antibody or a polyclonal antibody, or a fragment thereof) used as the secondary antibody is labeled preferably with a non-radioactive label. Enzyme labels, fluorescent labels, light emission labels, etc. can be used as the non-radioactive label. It is preferred that an enzyme label such as alkaline phosphatase, β-galactosidase or horse radish peroxidase be used.

In one aspect of the invention, at least two samples, or at least 3 samples, or at least 4 samples, or at least 5 samples obtained from the same individual at different points of time are tested for the presence or the amount of sFRP4. This may include collecting data over a period of time. Samples from a patient may be taken at regular intervals. The interval may range from about 2 to about 12 months, preferably it ranges from about 4 to about 8 months, more preferably it ranges from about 5 to about 7 months (e.g. about 6 months). This allows the monitoring of the progression of PKD over various periods of time.

Further disclosed herein is the use of a means for the detection of an sFRP (e.g. of sFRP4 or sFRP5) or its expression for diagnosing or monitoring the progression of PKD. Suitable means for the detection of an sFRP include but are not limited to antibodies against sFRP, nucleic acid molecules capable of hybridizing to a nucleic acid encoding sFRP, and the like. The antibodies according to this embodiment correspond to those described supra.

Suitable nucleic acid molecules (DNA or RNA) capable of hybridizing with a nucleic acid encoding sFRP4 or sFRP5 can be designed by one of ordinary skill using the published nucleotide sequence of the sFRP4 gene (Abu-Jawdeh G, Comella N, Tomita Y, Brown LF, Tognazzi K, Sokol SY, Kocher O. Differential expression of frpHE: a novel human stromal protein of the secreted frizzled gene family, during the endometrial cycle and malignancy.Lab Invest. 1999 Apr;79(4):439-47.). Usually suitable nucleic acid molecules have a length of at least ten nucleotides, preferably of at least 15 nucleotides, more preferably of at least 20 nucleotides, even more preferably of at least 25 nucleotides. The nucleic acid molecule may be about 15 to 35 nucleotides in length. The nucleic acid molecule may be used as a primer in a polymerase chain reaction (PCR) or as a probe in a hybridization assay. A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are well known and exemplified in Sambrook, J., Fritsch, E. F. and Maniatis, T. Molecular Cloning: A Laboratory Manual, Second Edition, Cold Spring Harbor Laboratory Press, Cold Spring Harbor (1989), particularly Chapter 11 therein. A specific example of stringent hybridization conditions is overnight incubation at 42° C in a solution comprising: 50% formamide, 5xSSC (150 mM NaCl, 15 mM trisodium citrate), 50 mM sodium phosphate (pH7.6), 5xDenhardt's solution, 10% dextran sulfate, and 20 micrograms/ml of denatured, sheared salmon sperm DNA, followed by washing the hybridization support in 0.1xSSC at about 65° C. In one aspect of the invention, RT-PCR could be used to monitor expression of sFRP4 in tubular epithelial cells present in the urine of patients with cystic renal disease. This disclosure described with respect to sFRP4 applies to other sFRPs such as sFRP1, sFRP2, sFRP3 and sFRP5.

Another aspect of the invention is the use of sFRP4 as a marker for the progression of PKD according to claim 9. The preferred embodiments of this aspect correspond to the preferred embodiments of the method described supra.

Yet another aspect of the present invention is the use of a diagnostic kit comprising at least one antibody capable of specifically binding to sFRP4 in a method of the present invention. Preferably, the test kit is an ELISA test kit. In one embodiment, the antibody does not crossreact with other proteins present in human urine. The preferred antibodies of this kit have been described supra in connection with the method of the invention. In one embodiment, the antibody in the test kit recognizes sFRP4 both in its native and in its denaturated form. In another embodiment, the antibody in the kit recognizes sFRP4 in the sample in an ELISA assay and on Western blots. In another embodiment, the binding capacity or specificity of the antibody is not significantly dependent on the glycosylation state of sFRP4. In another embodiment, the antibody recognizes sFRP4 in human urine or human serum in an ELISA assay (e.g. a typical sandwich ELISA). The antibody may be immobilized on a solid phase such as a microtiter plate or a test strip. The kit may comprise a further antibody capable of specifically binding to sFRP4. Usually, the second antibody is directed against the same protein as the first antibody (especially in case of an ELISA test kit). This second antibody may be used in a sandwich ELISA as known per se in the art. The second antibody (a monoclonal or a polyclonal antibody) may be labeled with a non-radioactive label. Enzyme labels, fluorescent labels, light emission labels, etc. can be used as the non-radioactive label. It is preferred that an enzyme label such as alkaline phosphatase, β-galactosidase or horse radish peroxidase be used. In one specific embodiment, the kit does not contain the antibody mAb 3.1 or 3.4 mentioned in Berndt et al. (2003). It is also possible that the second antibody recognizes the first antibody in the test kit, or that the second antibody recognizes an sFRP other than the sFRP recognized by the first antibody in the test kit.

The diagnostic kit may further comprise other components such as a wash buffer concentrate, a composition comprising sFRP4 as a standard, a stop solution (e.g. HCl solution) and the like. The composition comprising sFRP4 as a standard usually comprises substantially pure sFRP4. The kit may further comprise information material regarding the use of the kit, e.g. in the form of a sheet, a leaflet or a booklet. The information material may provide information on the kit materials, the sample preparation and/or the assay procedure. In particular, the information material may contain directions as to how the urine sample has to be prepared for the assay to be carried out. Preferably, the parts contained in the kit are arranged in a container which can be closed.

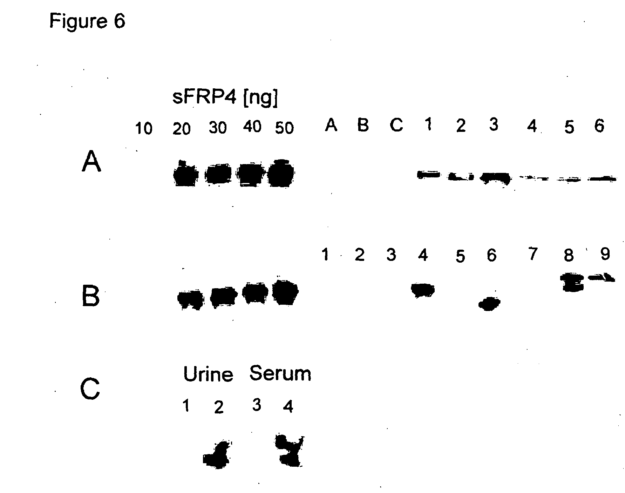

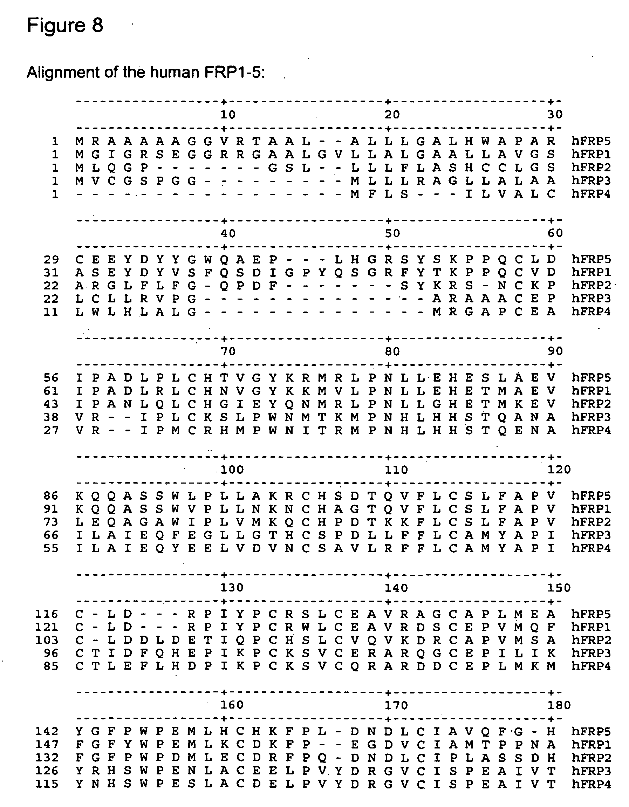

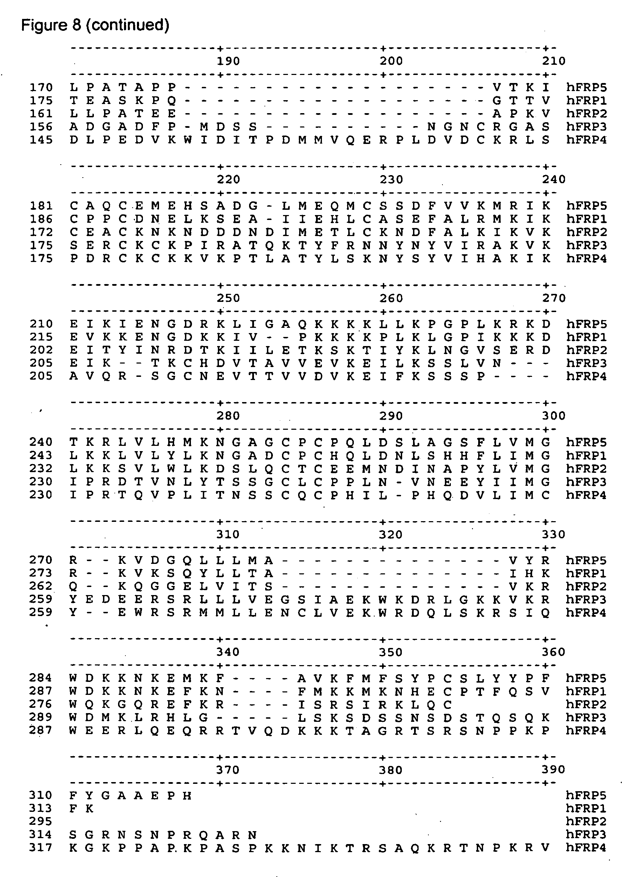

The present disclosure further relates to members of the sFRP gene family (see Figures 7 and 8). Currently, at least five closely related sFRP proteins have been described. All of these proteins are expressed in the kidney, and are potentially upregulated in PKD. As shown in Figure 6, several different species of secreted frizzled releated proteins are present in the urine of patients with PKD. All five sFRP proteins contain overlapping conserved protein sequences (see Figure 8: Alignment of sFRP1-5). Hence, another aspect of the present disclosure is the development of pan-reactive monoclonal antibodies and detection system(s) (as described above) that detect all five (and potentially more) secreted frizzled-related proteins and/or defined combination of sFRP proteins. In some instances (for example to detect subtypes of polycystic kidney diseases or other diseases associated with expression of sFRP proteins), it might also be useful to develop monoclonal antibodies and detection kits that specifically recognize sFRP1, sFRP2, sFRP3, or sFRP5.

The present disclosure relates to a screening method for identifying compounds effective in the treatment of PKD, comprising (a) contacting a test compound with a cell; and (b) determining the expression by the cell of one or more genes that are induced by cyst fluid or upregulated in kidneys of ADPKD patients, or determining the promoter activity of the promoter(s) of said gene(s).

The genes induced by cyst fluid or upregulated in kidneys of ADPKD patients may be selected from the genes listed in table 1 infra. In these screening methods the expression or promoter activity of at least 2, or at least 5, or at least 10, or at least 20, or at least 50 genes listed in table 1 may be determined.

The present disclosure further relates to a screening method for identifying compounds effective in the treatment of PKD, comprising (a) contacting a test compound with a cell; and (b) determining the expression by the cell of sFRP4 or sFRP5 (and/or other sFRP proteins), or determining the promoter activity of the sFRP4 or sFRP5 promoter (or other sFRP promoter) in the cell.

Suitable cells that may be used in the screening methods include, but are not limited to HEK 293 cells, COS, HeLa, etc.

The screening method may further comprise the step of selecting the test compound, if the test compound inhibits the expression by the cell of at least one sFRP or the promoter activity of the promoter of at least one sFRP. By way of example, the screening method may further comprise the step of selecting the test compound, if the test compound inhibits the expression by the cell of sFRP4 or the promoter activity of the sFRP4 promoter. Methods for determining the level of expression or the promoter activity are known to those of skill in the art and described in Wong et al. (2003).

A test compound may be regarded as inhibiting the expression by the cell of the sFRP, if the expression by the cell of the sFRP in the presence of the test compound is reduced by at least 10%, preferably at least 20%, more preferably at least 30%, even more preferably at least 40%, still more preferably at least 50%, most preferably at least 75%, relative to the expression by the cell of the sFRP in the absence of the test compound.

A test compound may be regarded as inhibiting the expression by the cell of sFRP4, if the expression by the cell of sFRP4 in the presence of the test compound is reduced by at least 10%, preferably at least 20%, more preferably at least 30%, even more preferably at least 40%, still more preferably at least 50%, most preferably at least 75%, relative to the expression by the cell of sFRP4 in the absence of the test compound.

A test compound may be regarded as inhibiting the expression by the cell of sFRP5, if the expression by the cell of sFRP5 in the presence of the test compound is reduced by at least 10%, preferably at least 20%, more preferably at least 30%, even more preferably at least 40%, still more preferably at least 50%, most preferably at least 75%, relative to the expression by the cell of sFRP5 in the absence of the test compound.

The embodiments described herein with respect to PKD can be applied to other renal disorders or other cystic kidney diseases. The embodiments described herein with respect to sFRP4 can be applied to other members of the sFRP family of proteins.

The present disclosure further relates to the following aspects:
(1) A method for diagnosing or monitoring the progression of polycystic kidney disease (PKD), comprising detecting a secreted frizzled related protein (sFRP) in a sample from an individual suffering from PKD or suspected of suffering from PKD.
(2) A method according to item (1), wherein the sample contains urine or serum obtained from the individual suffering from PKD or suspected of suffering from PKD.
(3) A method according to item (1) or (2), wherein the detection of an elevated level of the sFRP in the sample from the individual relative to control samples indicates a diagnosis of PKD, or wherein the extent of elevation of the sFRP in the sample from the individual relative to control samples correlates to the progression of PKD in the individual.
(4) A method according to any one of items (1) to (3), wherein the detection comprises contacting the sample with at least one antibody capable of binding to the sFRP to allow the formation of antibody-antigen complexes and detecting the presence of antibody-antigen complexes formed.
(5) A method according to any one of items (1) to (4), wherein the method comprises at least one direct or indirect immunoassay selected from the group consisting of a competitive binding assay, a non-competitive binding assay, a radioimmunoassay, an enzyme-linked immunosorbent assay (ELISA), a sandwich assay, a precipitin reaction, a gel diffusion immunodiffusion assay, an agglutination assay, a fluorescent immunoassay, chemiluminescence immunoassay, immunoPCR immunoassay, a protein A or protein G immunoassay, and an immunoelectrophoresis assay.
(6) A method according to any one of items (1) to (5), wherein at least two samples obtained from the same individual at different times are screened for the presence of the sFRP.
(7) A method according to any one of items (1) to (6), wherein said sFRP is secreted frizzled related protein-4 (sFRP4).
(8) The use of means for the detection of a secreted frizzled related protein (sFRP) for diagnosing or monitoring the progression of PKD.
(9) The use according to item (8), wherein the means for the detection of sFRP is an antibody capable of binding to the sFRP.
(10) The use of a secreted frizzled-related protein (sFRP) as a marker for the progression of PKD.
(11) The use according to any one of items (8) to (10), wherein said sFRP is secreted frizzled related protein-4 (sFRP4).
(12) A diagnostic kit for diagnosing or monitoring the progression of PKD, comprising at least one antibody capable of specifically binding to one or more secreted frizzled related proteins (sFRPs).
(13) A diagnostic kit according to item (12), wherein the antibody does not crossreact with other proteins present in human urine.
(14) A diagnostic kit according to item (12), wherein the antibody is capable of binding to sFRP1, sFRP2, sFRP3, sFRP4 and sFRP5.
(15) A diagnostic kit according to any one of items (12) to (14), wherein the antibody is immobilized on a solid phase.
(16) A diagnostic kit according to item (15), wherein the solid phase is a test strip.
(17) A diagnostic kit according to any one of items (12) to (16), further comprising a second antibody capable of specifically binding to said one or more sFRPs.
(18) A diagnostic kit according to any one of items (12) to (17), further comprising a wash buffer concentrate and/or a composition comprising sFRP.
(19) A screening method for identifying compounds effective in the treatment of PKD, comprising
   (a) contacting a test compound with a cell; and
   (b) determining the expression by the cell of an sFRP or determining the promoter activity of the promoter of an sFRP.
(20) A screening method according to item (19), further comprising the step of selecting the test compound if the test compound inhibits the expression by the cell of sFRP or the promoter activity of the sFRP promoter.

**Figure 1**
   A. sFRP4 expression in normal kidneys and tissue from patients with ADPK. The upper part depicts the relative expression obtained by microarray analysis. B. Semiquantitative RT-PCR was performed to confirm the microarray data (lower part). C. sFRP4 immunoreactivity in normal and ADPKD kidneys. Note that sFRP4 is present in some but not all tubular epithelial cells lining the depicted cysts. D. Cyst fluid stimulates expression of sFRP4 in differentiated IMCD cells (5-day culture) (left panel), but not in senescent IMCD cells (10-day culture) (right panel). E. Cyst fluid stimulates sFRP4 in differentiated proximal tubular (PT) cells (5-day culture) (left panel), but not in senescent PT cells (10-day culture) (right panel).
**Figure 2**
   A. Cyst fluid from patients with ADPKD stimulates the sFRP4 promoter. B. Two sFRP4 promoter fragments, -238/+83 and 1417/+83, are activated by cyst fluid, while the fragment -144/+83 shows little response to cyst fluid. C. Cyst fluid of ADPKD patients contain large amounts of sFRP4. Cyst fluid was diluted as indicated and compared to recombinant sFRP4 to estimate the concentration. In some cysts, sFRP4 concentrations as high as 1 mg/ml were observed. D. The molecular weight of sFRP4 in cyst fluid is reduced by F-deglykosidase, indicating that the secreted sFRP4 is highly glycosylated. E. Cyst fluid blocks XWnt8-stimulated activation of the TOPFLASH reporter, suggesting that sFRP4 in cyst fluid retains its biological activity (left panel). Cyst fluid has no effect on disheveled-mediated TOPFLASH activation (right panel). F. Partial purification of cyst fluid reveals that the Xwnt8-inhibiting activity correlates with the immunoreactive fractions.
**Figure 3**
   A. Microarray analysis separates IMCD treated with control media (n=3), and media containing 5 µg/ml recombinant sFRP4 (left panel: hierarchical clustering and dendogram; right panel: heatmap). B. GASP1 (left panel) and GASP9 (right panel) in IMCD cells treated either with control or with sFRP4-containing medium. The data of the left panel is derived from the microarray analysis, the right panel was generated using conditioned media from sFRP4-expressing HEK 293T cells. IMCD cells were either incubated with media of MOCK-transfected cells, or with the conditioned media for 24 hours. Semiquantitiate RT-PCR was performed, and the GASP9 signal was normalized for GAPDH expression. C. sFRP4 is excreted in the urine of patients with ADPKD. D. sFRP4 expression is upregulated in PKD2-deficient mice. RT-PCR and Western blot analysis was performed at E21 of either PKD2 (+/-) and PKD2 (-/-) mice. As shown in the left panel, there is a strong increase of sFRP4 in embryos, lacking PKD2. E. sFRP4 immunoreactivity is found in epithelial cells, lining the cysts of PKD2 (-/-) mice (left panel), while little sFRP4 can be found in wild-type mice. (right panel). F. INV mice revealed a similar upregulation of sFRP4, demonstrating that excessive sFRP4 production is not limited to the ADPKD, but likely accompanies cyst formation.
**Figure 4**
   A. Upregulation of sFRP4 detected by RT-PCR 5 and 10 days following 5/6 nephrectomy of mice. B. The graph depicts the average of sFRP4 expression detected by RT-PCR and normalized for β-gal of two independent experiments, demonstrated that 5/6 nephrectomy induces sFRP4 expression.
**Figure 5**
   A. The -144/+83 sFRP4 fragment is not activated by either Gα subunits or Disheveled. B. The -238/+83 sFRP4 fragment can be activated by both Ga subunits and Dishevel. Since the short sFRP4 fragment does not contain a TCF-binding site, Wnt-mediated activation is likely mediated in part through G-protein dependent signaling.
**Figure 6**
   A. Detection of sFRP4 in the urine of ESRD patients with cystic kidney disease in comparison to recombinant human sFRP4 (ng per lane as indicated). Sample A, B, C are control urines from healthy volunteers. No. 1-6 are urine samples from ESRD patients with either ADPKD, or acquired cystic kidney disease.
   B. Detection of sFRP4 in the urine of patients with ADPKD and various degrees of renal failure in comparison to recombinant human sFRP4 (ng per lane as indicated). Note the different molecular weight sizes, indicating that patients with ADPKD secrete several closely related sFRP proteins.
   C. Detection of sFRP4 in the blood of patients with ADPKD. Lane 1 and 3 are urine and serum of a healthy volunteer, lane 2 and 4 are the urine and serum of a patient with ADPKD. To detect sFRP4 in the serum, sFRP4 was immunoprecipitated from 50 µl serum, using an sFRP4-specific polyclonal antiserum. The precipitated protein was subsequently detected by Western blot analysis.
**Figure 7** shows the amino acid sequences of sFRP1, sFRP2, sFRP3, sFRP4, and sFRP5. Via the accession numbers indicated, further information on the sFRPs can be obtained. In particular, the nucleic acid sequences encoding the sFRPs can be retrieved (e.g. from the indicated publication, or by way of a link to the cDNA sequence), allowing the design of suitable primers and probes.
**Figure 8** shows an alignment of the amino acid sequences of sFRP1, sFRP2, sFRP3, sFRP4, and sFRP5. The alignment shows conserved regions and unique regions of the sFRPs.

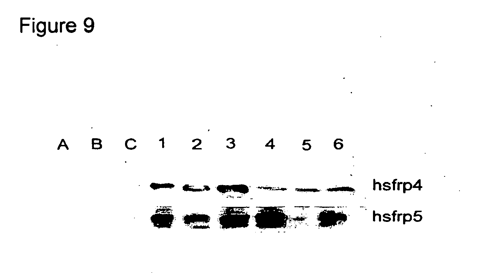

**Figure 9** depicts the detection of human sFRP4 and sFRP5 in the urine of patients with polycystic kidney disease (PKD). Urine of healthy controls (A-C) do not contain detectable amounts of sFRP4 or sFRP5 in the urine. In contrast, PKD patients (1- 6) excrete elevated amounts of sFRP4 and sFRP5.

The following non-limiting examples further illustrate the invention. It should be understood, however, that the invention as claimed should not be unduly limited to such specific embodiments.

### Methods

**Materials**. Patient samples (discarded material, serum, and urine) were obtained after approval of the study protocol by the local ethics committee. Kidney tissue specimen were either snap-frozen in liquid nitrogen for RNA isolation, or immediately fixed in 4% paraformaldehyde for immunohistochemistry. Cyst fluid was collected by aspiration, and stored at either 4°C (short-term) or -20°C (long-term). Blood samples were collected in collecting tubes, and centrifuged at 4°C for 10 min at 1200 rpm to obtain serum. Serum and urine were stored at -20°C (long-term). For RNA isolation, frozen tissue was homogenized in 4 M guanidinium isothiocyanate/0.72%β-mercaptoethanol using a polytron (PT-MR2100, Kinematica AG, Lucerne, Switzerland). Total RNA was subsequently purified on a caesium chloride gradient. The sFRP-4 monoclonal antibody was recently described (1).

**Gene expression profiling and semiquantative RT-PCR**. Microarray analysis was performed as recently described (2). For semi quantitative RT-PCR, total RNA (1µg) was purified with the RNase free DNase set (Qiagen, Hilden, Germany), and reverse transcribed into cDNAs using an oligo (dT) 12-18 primer and Superscript II reverse transcriptase (Invitrogen, Carlsbad, CA, USA). For each PCR reaction, β-actin or GAPDH expression was used as an internal standard control (22). The following primers were used for sFRP4 amplification - 5' atcggtgcaagtgcaaaaag 3' (SEQ ID NO:8) and 5' tatgtggacactggcaggag 3' (SEQ ID NO:9), for GASP2 amplification -5' cttcaggtgtacatgaataaa 3' (SEQ ID NO:10) and 5' gaggtcgtggtgatttgctaaggc 3' (SEQ ID NO:11) and GASP9 amplification - 5' gcctctgctaatggcggacaggc 3' (SEQ ID NO:12) and 5' gggaggcctcccaattggcgg 3' (SEQ ID NO:13), respectively. The amplification cycle consisted of a hot start at 94°C for 2 min., followed by multiple cycles of denaturation at 94°C at 1 min., annealing at 58°C for 1 min and elongation at 72°C for 1 min. Cycle number was adjusted to mRNA expression level of the different genes. The PCR products were separated on a 2% agarose gel and analyzed in relation to the corresponding control band.

**Cell culture**. Conditionally immortalized proximal tubular epithelial (PT) cells from the SV40 immorto mouse (kindly provided by H. Pavenstädt, Münster) were maintained at 33°C in DMEM-F12 supplemented with 2% calf serum. For differentiation of the PT cells, temperature was raised to 37°C. Immortalized mIMCD-3 cells (referred to as IMCD cells)

(ATCC) were grown in DMEM- F12 medium supplemented with 10% calf serum. Human embryonic kidney cells (referred to as HEK-293T cells) were grown in DMEM supplemented with 10% calf serum.

**Western blot analysis**. Immunoprecipitation and Western blot analysis were performed as described (3).

**Immunochemistry**. Tissue samples obtained from patient materials were fixed in 4% paraformaldehyde, dehydrated and paraffin embedded. Tissue sections (5 µ) were stained, using the Vectastain-ABC kit (Vector Labortories). Mice embryos were prepared, using the Technovit 7100 embedding-kit (Heraeus-Kulzer, Germany). All sections were examined using a Zeiss microscope.

**Reporter Assays**. HEK 293T cells were seeded into 12-well plates 24 hours prior to transfection. To determine luciferase or alkaline phosphatase activity, 2 µg TOPFLASH or pSEAP2B reporter constructs were transfected with 1 µg β-galactosidase plasmid, using the calcium phosphate method. Transfections were performed in triplicate. After 24 hours, cell lysates and supernatants were assayed for luciferase and alkaline phosphatase activity, respectively (Great EscAPe SEAP, BD Bioscience). The results are presented as relative luciferase or alkaline phosphatase activity, normalizated for β-galactosidase activity.

**Column Chromatography**. Cystfluid containing sFRP4 was concentrated at 5000 xg for 45 min, using a YM-30 Centricon Centrifugal Filter Device, followed by preclearing at 45000 RPM and 4°C for 30 minutes. Concentrated cyst fluid was dialysed overnight at 4°C against equilibration buffer (20mM NaH₂PO₄, 30 mM NaCl, pH 7.5). One ml of the dialyzed cyst fluid was loaded onto a HighPrep 16/10 SP-FF Sephadex-Cation exchange column. After washing the column with 58 ml equilibration buffer, elution was carried out with a continuous gradient starting with 20mM NaH₂PO₄, 30 mM NaCl, pH 7.5, and ending with 20mM NaH₂PO₄, 1M NaCl, pH 7.5. During elution, 2 ml fractions were collected, and dialyzed overnight at 4°C against PBS. Fractions were tested for the presence of sFRP4 by Western blot analysis.

### Results

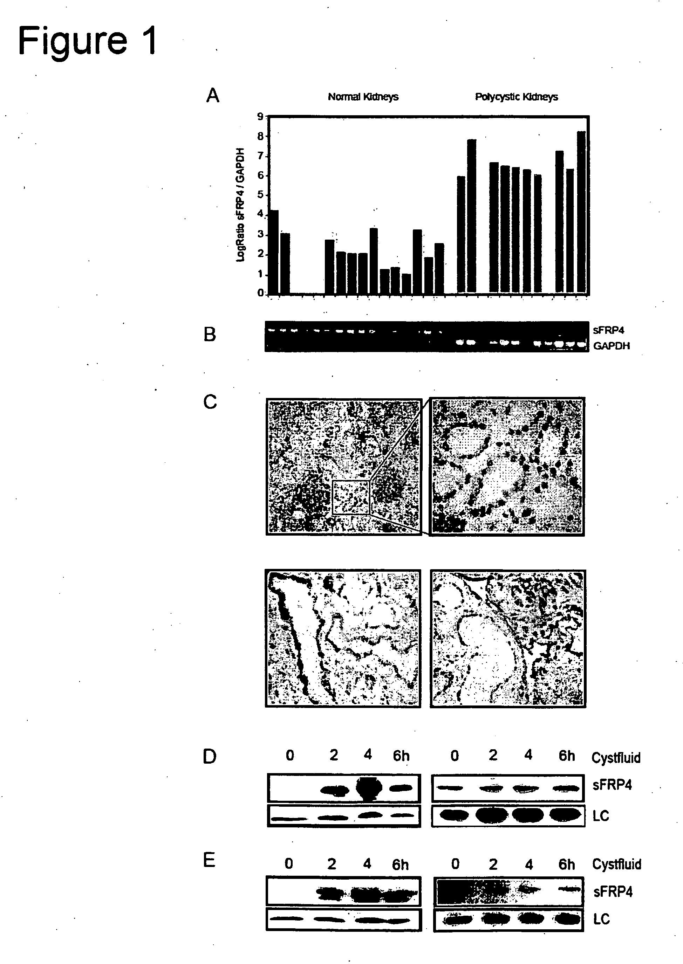

To understand the molecular mechanism associated with ESRD in ADPKD, we generated gene profiles of ADPKD kidneys, and identified sFRP4 as a differentially regulated gene (Fig. 1a). sFRP4 (secreted frizzled-related protein-4) is a member of an evolving family of soluble molecules that bind both Wnt and frizzled molecules (4). RT-PCR confirmed that most ADPKD kidneys expressed increased amounts of sFRP4 (Fig. 1b). Immunoreactive sFRP4 was detectable in tubular epithelial cells lining the cysts in ADPKD kidneys, while tubular epithelial cells of normal kidneys expressed no sFRP4 (Fig. 1c). sFRP4 was not uniformly expressed in all cyst epithelial cells, and *in vitro* experiments using IMCD and MT1 cells, two tubular epithelial cell lines, suggested that cyst fluid triggers sFRP4 in less-than terminally differentiated cells (Figs. 1d and 1e).

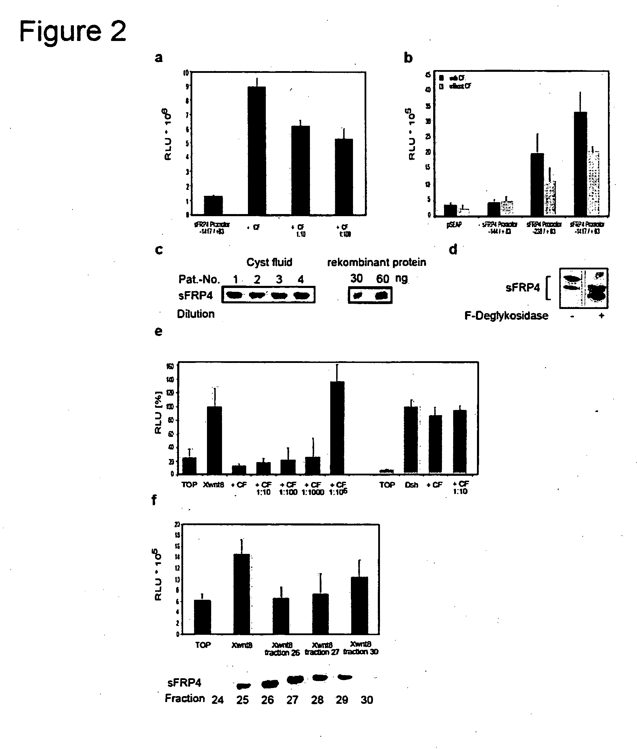

Cyst fluid contains a lipophilic substance that stimulates generation of cAMP (5), as well as several growth factors and hormones such as vasopression. Since there are two cAMP-responsive elements present in the sFRP4-promoter (Fig. 2a) (6), we analyzed the cyst fluid-mediated activation of different sFRP4 promoter fragments. As shown in Fig. 2b, cyst fluid significantly activated the sFRP4 promoter -238/83 and -1417/83, but failed to stimulate sFRP4 -144/83 lacking the cAMP-responsive elements. Thus, the previously described cAMP-stimulating activity of cyst fluid seems to stimulate sFRP4 gene expression.

Since sFRP4 is a secreted protein, we tested whether this molecule accumulates in the cyst fluid. Western blot analysis revealed that cyst fluid contains large amounts of highly glycosylated sFRP4 (Figs. 2c and 2d). sFRP4 blocks canonical Wnt signaling, and prevents XWnt8-mediated double axis formation during early *Xenopus* development. As shown in Fig. 2e, cyst fluid, containing large amounts for sFRP4, very effectively blocked XWnt8-mediated activation of the Tcf-defendant TOPFLASH-reporter construct, but had very little effect on the cytoplasmic Wnt activator molecule disheveled (dsh). We partially purified sFRP4 to further demonstrate that cystic sFRP4 retains its bioactivity. As shown in Fig. 2e, fractions containing immunoreactive sFRP4 blocked XWnt8-mediated TOPFlash activation, while sFRP4-negative fractions had no significant effect. Taken together, these findings show that cysts contain large amounts of biologically active sFRP4. Several reports have linked increased expression of sFRP4 to accelerated apoptosis rates. Although the mechanism of sFRP4-induced apoptosis are unknown, increased sFRP4 expression in ADPKD coincides with increased apoptosis of the surrounding normal renal tissue, indicating that cystic sFRP4 may contribute to the progression of renal failure.

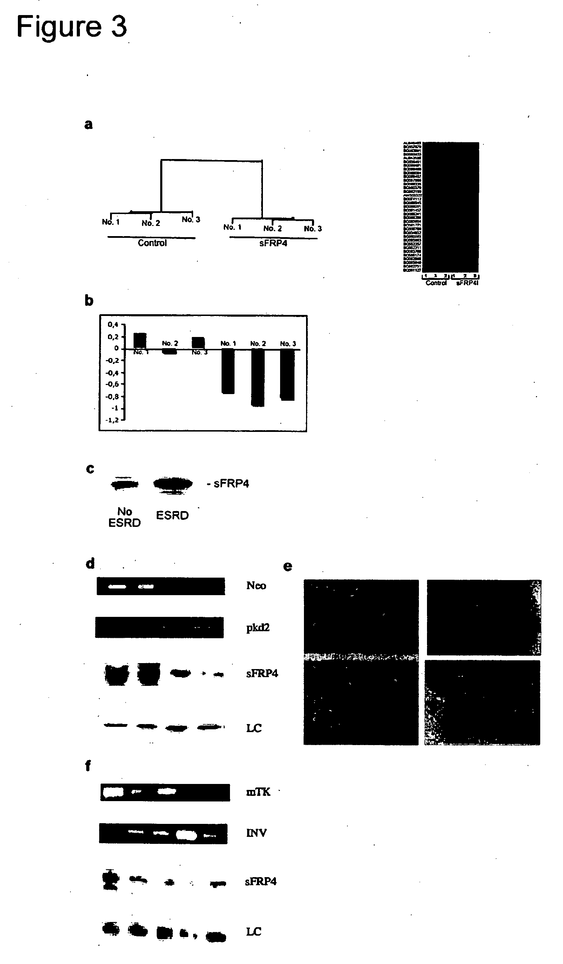

To understand the effects of sFRP4 on renal tubular epithelial cells, we incubated IMCD cells with recombinant sFRP4, and determined the genes differentially regulated in sFRP4-treated cells by microarray analysis, using a 22.5 k murine cDNA chip. As demonstrated in Fig. 3a, sFRP4 treatment separated the gene profiles of the two culture sets, but yielded only a few significantly regulated genes (FDR < 0.05 and p < 0.05). One potential target of sFRP4 treatment was a GASP family member. To confirm differential regulation of GASP, we treated IMCD cells with sFRP4-conditioned media, and observed down-regulation of GASP9, a renal-specific GASP family member (Fig. 3b). GASP proteins mediate the endocytosis of G-protein coupled receptors (GPCR), while the lack of GASP proteins causes accumulation of GPCRs at the cell surface. Thus, persistent exposure to sFRP4 could establish a detrimental feedback loop, in which cyst fluid activates sFRP4 that in turn prevents endocystosis of GPCRs through downregulation of GASP proteins.

Cysts typically loose their connection to the draining tubules. Nevertheless, we detected sFRP4 in the urine of patients with ADPKD. Fig. 3c shows that the extent of sFRP4 excretion correlates with stage of renal failure. These findings suggest that urinary sFRP4 excretion is a potential marker to monitor progression of renal disease in patients with ADPKD.

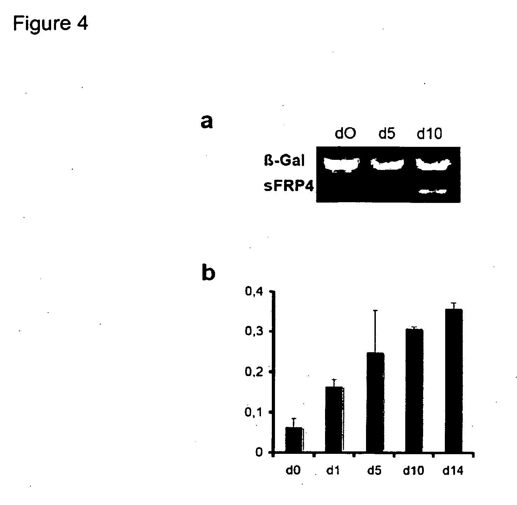

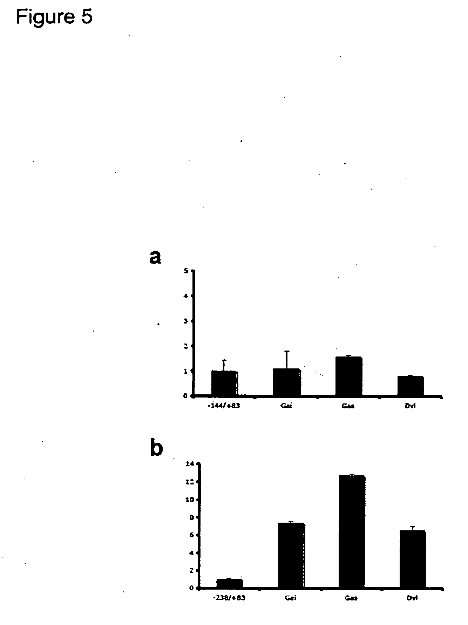

Since *PKD2* is mutated in some patients with ADPKD, we examined sFRP4 expression in *PKD2* (-/-) mice. Typically homocygote animals die between E15 and E20 of embryogenesis. As shown in Figs. 3d and 3e, increased levels of sFRP4 are detectable in *PKD2* (-/-), but not in *PKD2* (+/-) or in wild-type mice. To determine whether sFRP4 upregulation is limited to ADPKD, we performed similar experiments in homocygote INV mice. These mice are deficient in NPH2 (Inversin), a gene mutated in autosomal recessive nephronophthisis type II (infantile form). INV mice revealed a similar upregulation of sFRP4, demonstrating that excessive sFRP4 production is not limited to the ADPKD, but likely accompanies cyst formation (Fig. 3F). Since increased sFRP4 is also detectable after partial nephrectomy (see Figure 4), we postulate that sFRP4 expression is stimulated by compounds secreted into the cyst fluid, but are also produced during regenerative responses.

Taken together, our findings reveal that cyst formation is associated with increased production and accumulation of sFRP4, a frizzled-related Wnt antagonist implicated in increased apoptosis. sFRP4 downregulates GASP proteins in tubular epithelial cells, and thus, could confer increased susceptibility to ligands that bind and stimulate GPCRs. Since ADPKD is a slowly progressive disease, the success of therapeutic interventions has been difficult to monitor. Since sFRP4 is excreted in the urine of patients with ADPKD, it may represent a novel marker for disease progression, and help to monitor the response to therapeutic interventions aimed to slow cyst development.

In an additional microarray analysis, using a 38 k human cDNA chip, additional molecules that are highly upregulated in ADPKD kidney were identified. The gene products, in particular proteins and fragments thereof, of the genes upregulated in ADPKD are useful to detect or monitor patients with PKD.

**Table 1: Significantly regulated genes in ADPKD. The listed genes are significantly upregulated in ADPKD kidneys. Fold increase is expressed as log 2. Peptides of genes shaded in grey have been identified in the urine of healthy individuals.**

| **Gene** | **Description** | **Gene** **Symbol** | **Fold** **Increase** **(log 2)** |
|---|---|---|---|
| H25590 | Serum amyloid A1 | SAA1 | 8,825 |
| N28788 | Thrombospondin 2 | THBS2 | 8,418 |
| H47153 | Collagen, type VI, alpha 3 | COL6A3 | 7,828 |
| R46444 | AE binding protein 1 | AEBP1 | 5,91 |
| CR746674 | Tubulin, alpha 3 | TUBA3 | 5,674 |
| BX093514 | Transgelin | TAGLN | 5,618 |
| R72711 | Synaptopodin 2 | SYNP02 | 5,502 |
| H58645 | Anthrax toxin receptor 1 | ANTXR1 | 5,341 |
| W86529 | Microfibrillar associated protein 5 | MFAP5 | 5,292 |
| AA011468 | Collagen, type XIV, alpha 1 (undulin) | COL14A1 | 5,258 |
| W57893 | Fibronectin 1 | FN1 | 5,045 |
| T97662 | Sushi, von Willebrand factor type A, EGF and pentraxin domain containing 1 | SVEP1 | 4,27 |
| R97974 | CD53 molecule | CD53 | 4,253 |
| R22873 | Cathepsin S | CTSS | 4,18 |
| BX110513 | Integrin, beta 6 | ITGB6 | 4,176 |
| W40296 | Latent transforming growth factor beta binding protein 1 | LTBP1 | 4,076 |
| H77318 | Methionine sulfoxide reductase B3 | MSRB3 | 4,051 |
| BX101810 | Hypothetical protein FLJ21986 | FLJ21986 | 4,048 |
| R21642 | Fibulin 1 | FBLN1 | 4,034 |
| H64964 | Membrane-spanning 4-domains, subfamily A, member 6A | MS4A6A | 3,859 |
| H69049 | CD36 molecule (thrombospondin receptor) | CD36 | 3,837 |
| AA029988 | Sulfatase 1 | SULF1 | 3,815 |
| BX115823 | Protease, serine, 23 | PRSS23 | 3,715 |
| BX092184 | Secreted frizzled-related protein 4 | SFRP4 | 3,7 |
| T95571 | Collagen, type XII, alpha 1 | COL12A1 | 3,465 |
| N78528 | EGF-like repeats and discoidin I-like domains 3 | EDIL3 | 3,396 |
| R27079 | Lipoma HMGIC fusion partner | LHFP | 3,353 |
| R06034 | Pleckstrin | PLEK | 3,205 |
| R82964 | Fatty acid binding protein 4, adipocyte | FABP4 | 3,121 |
| BX090914 | Aldehyde dehydrogenase 1 family, member A3 | ALDH1A3 | 3,089 |
| BX279620 | Polo-like kinase 2 (Drosophila) | PLK2 | 3,063 |
| R72689 | Myosin, heavy polypeptide 11, smooth muscle | MYH11 | 3,036 |
| AA134668 | Four and a half LIM domains 1 | FHL1 | 2,866 |
| T70850 | Perilipin | PLIN | 2,776 |
| BX283933 | Myosin-reactive immunoglobulin heavy chain variable region | LOC652106 | 2,647 |
| BG104623 | Nephroblastoma overexpressed gene | NOV | 2,636 |
| H67479 | Biglycan | BGN | 2,575 |
| T89391 | Caveolin 2 | CAV2 | 2,557 |
| H46619 | Myosin, light polypeptide 9, regulatory | MYL9 | 2,526 |
| N44001 | SET domain containing (lysine methyltransferase) 7 | SETD7 | 2,501 |
| BX282272 | CDC42 effector protein (Rho GTPase binding) 3 | CDC42EP3 | 2,394 |
| BX093905 | Tripartite motif-containing 22 | TRIM22 | 2,345 |
| W79256 | Erythrocyte membrane protein band 4.1-like 2 | EPB41 L2 | 2,326 |
| BX114634 | Tropomyosin 4 | TPM4 | 2,287 |
| AA035192 | Carboxypeptidase X (M14 family), member 2 | CPXM2 | 2,257 |
| AA039256 | Asporin (LRR class 1) | ASPN | 2,228 |
| R83765 | Potassium channel tetramerisation domain containing 10 | KCTD10 | 2,221 |
| N57594 | Transmembrane protein 47 | TMEM47 | 2,2 |
| AA292054 | Growth arrest-specific 1 | GAS1 | 2,17 |
| H56349 | Fibrinogen-like 2 | FGL2 | 2,166 |
| AA027246 | Glycerol-3-phosphate acyltransferase, mitochondrial | GPAM | 2,164 |
| BX090875 | Pelota homolog (Drosophila) | PELO | 2,115 |
| BX114711 | Proteoglycan 1, secretory granule | PRG1 | 2,042 |
| BG697186 | Potassium voltage-gated channel, subfamily H (eag-related), member 2 | KCNH2 | 1,936 |
| H90971 | Poly (ADP-ribose) polymerase family, member 14 | PARP14 | 1,919 |
| R12367 | Proteasome (prosome, macropain) subunit, beta type, 8 (large multifunctional peptidase 7) | PSMB8 | 1,913 |
| H74106 | LIM domain binding 2 | LDB2 | 1,894 |
| H13471 | Syndecan 2 (heparan sulfate proteoglycan 1, cell surface-associated, fibroglycan) | SDC2 | 1,89 |
| BM921371 | Fibulin 5 | FBLN5 | 1,882 |
| R97862 | WD repeat and SOCS box-containing 1 | WSB1 | 1,881 |
| BX098913 | Signal transducer and activator of transcription 1, 91 kDa | STAT1 | 1,818 |
| R08415 | Sema domain, immunoglobulin domain (Ig), transmembrane domain (TM) and short cytoplasmic domain, (semaphorin) 4G | SEMA4G | 1,795 |
| BG679464 | Cysteine-rich, angiogenic inducer, 61 | CYR61 | 1,7 |
| R27042 | Wiskott-Aldrich syndrome protein interacting protein | WASPIP | 1,629 |
| BM473315 | N-myc (and STAT) interactor | NMI | 1,564 |
| AA054773 | GRINL1A combined protein | Gcom1 | 1,504 |

A method for diagnosing or monitoring the progression of a renal disorder might comprise detecting one or more of the gene products encoded by the genes listed in table 1 in a urine or serum sample from an individual suffering from the renal disorder or suspected of suffering from the renal disorder. The various aspects of this method correspond to those described for sFRP4 and sFRP5 herein *mutatis mutandis.* The method may comprise detecting at least one, at least two, at least 5, at least 10, at least 20 or at least 50 gene products encoded by the respective genes listed in table 1. The term "gene product" includes proteins and fragments thereof.

### References

1. Berndt T, Craig TA, Bowe AE, Vassiliadis J, Reczek D, Finnegan R, et al. Secreted frizzled-related protein 4 is a potent tumor-derived phosphaturic agent. J Clin Invest 2003;112(5):785-94.
2. Donauer J, Rumberger B, Klein M, Faller D, Wilpert J, Sparna T, et al. Expression profiling on chronically rejected transplant kidneys. Transplantation 2003;76(3):539-47.
3. Benzing T, Gerke P, Hopker K, Hildebrandt F, Kim E, Walz G. Nephrocystin interacts with Pyk2, p130(Cas), and tensin and triggers phosphorylation of Pyk2. Proc Natl Acad Sci U S A 2001;98(17):9784-9.
4. Jones SE, Jomary C. Secreted Frizzled-related proteins: searching for relationships and patterns. Bioessays 2002;24(9):811-20.
5. Yamaguchi T, Nagao S, Takahashi H, Ye M, Grantham JJ. Cyst fluid from a murine model of polycystic kidney disease stimulates fluid secretion, cyclic adenosine monophosphate accumulation, and cell proliferation by Madin-Darby canine kidney cells in vitro. Am J Kidney Dis 1995;25(3):471-7.
6. Wong VK, Yam JW, Hsiao WL. Cloning and characterization of the promoter region of the mouse frizzled-related protein 4 gene. Biol Chem 2003;384(8):1147-54.
7. Donauer J. et al., Expression profiling of polycystic kidney disease. Journal of the American Society of Nephrology 2003; vol. 14, Abstracts issue, page 106A.
8. Zheng D. et al., Urinary excretion of monocyte chemoattractant protein 1 in ADPKD. Journal of the American Society of Nephrology 2003; vol. 14, pages 2588 - 2595.

## Claims

1. A method for diagnosing or monitoring the progression of a polycystic kidney disease (PKD), comprising detecting a secreted frizzled related protein (sFRP) in a sample containing urine or serum obtained from an individual suffering from PKD or suspected of suffering from PKD, wherein said sFRP is secreted frizzled related protein-4 (sFRP4) having the amino acid sequence as shown in SEQ ID NO:4.

2. A method according to claim 1, wherein said sample is a urine sample.

3. A method according to claim 1, wherein said sample is a serum sample.

4. A method according to any one of the preceding claims, wherein the detection of an elevated level of the sFRP4 in the sample from the individual relative to control samples indicates a diagnosis of PKD, or wherein the extent of elevation of the sFRP4 in the sample from the individual relative to control samples correlates to the progression of PKD in the individual.

5. A method according to any one of claims 1 to 4, wherein the detection comprises contacting the sample with at least one antibody capable of binding to the sFRP4 to allow the formation of antibody-antigen complexes and detecting the presence of antibody-antigen complexes formed.

6. A method according to any one of claims 1 to 5, wherein the method comprises at least one direct or indirect immunoassay selected from the group consisting of a competitive binding assay, a non-competitive binding assay, a radioimmunoassay, an enzyme-linked immunosorbent assay (ELISA), a sandwich assay, a precipitin reaction, a gel diffusion immunodiffusion assay, an agglutination assay, a fluorescent immunoassay, chemiluminescence immunoassay, immunoPCR immunoassay, a protein A or protein G immunoassay, and an immunoelectrophoresis assay.

7. A method according to any one of claims 1 to 6, wherein at least two samples obtained from the same individual at different times are screened for the presence of the sFRP4.

8. A method according to any one of the preceding claims, which comprises detecting both sFRP4 and sFRP5 in said sample.

9. The use of a secreted frizzled-related protein (sFRP) in a sample containing urine or serum as a marker for the progression of PKD, wherein said sFRP is secreted frizzled related protein-4 (sFRP4) having the amino acid sequence as shown in SEQ ID NO:4.

10. The use of a diagnostic kit comprising at least one antibody capable of binding to sFRP4 in a method according to any one of claims 1 to 8, wherein said sFRP4 has the amino acid sequence as shown in SEQ ID NO:4.

11. The use according to claim 10, **characterized in that** said antibody is capable of binding to sFRP4 present in human urine.

12. The use according to claim 10 or 11, wherein the antibody does not crossreact with other proteins present in human urine.

13. The use according to claim 12, wherein the antibody is specific for sFRP4.

14. The use according to claim 10 or 11, wherein the antibody is capable of binding to sFRP1, sFRP2, sFRP3, sFRP4 and sFRP5.

15. The use according to any one of claims 10 to 14, wherein the antibody is immobilized on a solid phase.

16. The use according to claim 15, wherein the solid phase is a test strip.

17. The use according to any one of claims 10 to 16, wherein said kit further comprises a second antibody capable of specifically binding to sFRP4.

18. The use according to any one of claims 10 to 17, wherein said kit further comprises a wash buffer concentrate and/or a composition comprising sFRP4.

## Patentansprüche

1. Verfahren zur Diagnose oder Überwachung des Fortschreitens einer polyzystischen Nierenerkrankung (PKD), umfassend das Detektieren eines secreted Frizzled Related Proteins (sFRP) in einer Probe, enthaltend Urin oder Serum, erhalten von einem Individuum, das an PKD leidet oder mutmaßlich an PKD leidet, wobei das sFRP das secreted Frizzled Related Protein-4 (sFRP4) mit der in SEQ ID Nr. 4 gezeigten Aminosäuresequenz ist.

2. Verfahren nach Anspruch 1, wobei die Probe eine Urinprobe ist.

3. Verfahren nach Anspruch 1, wobei die Probe eine Serumprobe ist.

4. Verfahren nach einem der vorhergehenden Ansprüche, wobei die Detektion eines erhöhten Spiegels des sFRP4 in der Probe von dem Individuum bezogen auf Kontrollproben eine Diagnose von PKD anzeigt, oder wobei das Ausmaß der Erhöhung des sFRP4 in der Probe von dem Individuum bezogen auf Kontrollproben mit dem Fortschreiten von PKD in dem Individuum korreliert.

5. Verfahren nach einem der Ansprüche 1 bis 4, wobei die Detektion das Kontaktieren der Probe mit mindestens einem Antikörper, der an das sFRP4 binden kann, damit Antikörper-Antigen-Komplexe gebildet werden können, und das Detektieren der Gegenwart gebildeter Antikörper-Antigen-Komplexe umfasst.

6. Verfahren nach einem der Ansprüche 1 bis 5, wobei das Verfahren mindestens einen direkten oder indirekten Immunoassay, ausgewählt aus der Gruppe, bestehend aus einem kompetitiven Bindungsassay, nicht-kompetitiven Bindungsassay, einem Radioimmunoassay, einer enzymgekoppelten Immunadsorptionsbestimmung (ELISA), einem Sandwich-Test, einer Präzipitinreaktion, einem Geldiffusions-Immunodiffusions-Assay, einem Agglutinationstest, einem Fluoreszenzimmunoassay, einem Chemolumineszenzimmunoassay, einem ImmunoPCR-Immunoassay, einem Protein-A- oder Protein-G-Immunoassay und einem Immunoelektrophoreseassay, umfasst.

7. Verfahren nach einem der Ansprüche 1 bis 6, wobei mindestens zwei Proben, erhalten von demselben Individuum zu unterschiedlichen Zeiten, auf die Gegenwart des sFRP4 gescreent werden.

8. Verfahren nach einem der vorhergehenden Ansprüche, welches das Detektieren sowohl von sFRP4 als auch sFRP5 in der Probe umfasst.

9. Verwendung eines secreted Frizzled Related Proteins (sFRP) in einer Probe, enthaltend Urin oder Serum, als Marker für das Forschreiten von PKD, wobei das sFRP das secreted Frizzled Related Protein-4 (sFRP4) mit der in SEQ ID Nr. 4 gezeigten Aminosäuresequenz ist.

10. Verwendung eines Diagnosekits, umfassend mindestens einen Antikörper, der an sFRP4 binden kann, in einem Verfahren nach einem der Ansprüche 1 bis 8, wobei das sFRP4 die in SEQ ID Nr. 4 gezeigte Aminosäuresequenz aufweist.

11. Verwendung nach Anspruch 10, **dadurch gekennzeichnet, dass** der Antikörper an sFRP4, das in humanem Urin vorliegt, binden kann.

12. Verwendung nach Anspruch 10 oder 11, wobei der Antikörper nicht mit anderen Proteinen, die in humanem Urin vorliegen, kreuzreagiert.

13. Verwendung nach Anspruch 12, wobei der Antikörper spezifisch ist für sFRP4.

14. Verwendung nach Anspruch 10 oder 11, wobei der Antikörper an sFRP1, sFRP2, sFRP3, sFRP4 und sFRP5 binden kann.

15. Verwendung nach einem der Ansprüche 10 bis 14, wobei der Antikörper an einer festen Phase immobilisiert ist.

16. Verwendung nach Anspruch 15, wobei die feste Phase ein Teststreifen ist.

17. Verwendung nach einem der Ansprüche 10 bis 16, wobei das Kit ferner einen zweiten Antikörper umfasst, der spezifisch an sFRP4 binden kann.

18. Verwendung nach einem der Ansprüche 10 bis 17, wobei das Kit ferner ein Waschpufferkonzentrat und/oder eine Zusammensetzung, umfassend sFRP4, umfasst.

## Revendications

1. Procédé pour le diagnostic ou la surveillance de la progression d'une polykystose rénale (PKD), comprenant la détection d'une protéine à liaison grillée secrétée (sFRP) dans un échantillon contenant de l'urine ou du sérum, obtenu à partir d'un sujet souffrant de PKD ou suspecté de souffrir de PKD, dans lequel ladite sFRP est une protéine-4 à liaison grillée secrétée (sFRP4) possédant la séquence d'acides aminés illustrée dans l'ID de SEQ n°4.

2. Procédé selon la revendication 1, dans lequel ledit échantillon est un échantillon d'urine.

3. Procédé selon la revendication 1, dans lequel ledit échantillon est un échantillon de sérum.

4. Procédé selon l'une quelconque des revendications qui précèdent, dans lequel la détection d'un niveau élevé de la sFRP4 dans l'échantillon du sujet par rapport à des échantillons de contrôle indique un diagnostic de PKD ou dans lequel l'étendue de l'élévation de la sFRP4 dans l'échantillon du sujet par rapport aux échantillons de contrôle est en corrélation avec la progression de la PKD chez le sujet.

5. Procédé selon l'une quelconque des revendications 1 à 4, dans lequel la détection comprend le contact de l'échantillon avec au moins un anticorps capable de se lier à la sFRP4 pour permettre la formation de complexes anticorps-antigène et la détection de la présence de complexes anticorps-antigène formés.

6. Procédé selon l'une quelconque des revendications 1 à 5, dans lequel le procédé comprend au moins un immunodosage direct ou indirect sélectionné provenant du groupe comprenant un dosage de liaison compétitif, un dosage de liaison non compétitif, un radioimmunodosage, un dosage par la méthode ELISA, un dosage sandwich, une réaction de précipitine, un dosage d'immunodiffusion à diffusion de gel, un dosage d'agglutination, un immunodosage fluorescent, un immunodosage à chimioluminescence, un immunodosage immunoPCR, un immunodosage à protéine A ou protéine G et un dosage d'immunoélectrophorèse.

7. Procédé selon l'une quelconque des revendications 1 à 6, dans lequel au moins deux échantillons obtenus à partir du même sujet à des moments différents sont passés au crible quant à la présence de la sFRP4.

8. Procédé selon l'une quelconque des revendications qui précèdent, lequel comprend la détection à la fois de la protéine sFRP4 et de la protéine sFRP5 dans ledit échantillon.

9. Utilisation d'une protéine à liaison grillée secrétée (sFRP) dans un échantillon contenant de l'urine ou du sérum en tant que marqueur pour la progression de la PKD, où ladite sFRP est une protéine-4 à liaison grillée secrétée (sFRP4), ayant la séquence d'acides aminés illustrée dans l'ID de SEQ n°:4.

10. Utilisation d'un kit de diagnostic comprenant au moins un anticorps capable de se lier à la FRP4 dans un procédé selon l'une quelconque des revendications 1 à 8, où ladite sFRP4 possède la séquence d'acides aminés illustrée dans l'ID de SEQ n°:4.

11. Utilisation selon la revendication 10, **caractérisée en ce que** ledit anticorps est capable de se lier à la sFRP4 présente dans l'urine humaine.

12. Utilisation selon la revendication 10 ou 11, où l'anticorps n'a pas de réaction croisée avec d'autres protéines présentes dans l'urine humaine.

13. Utilisation selon la revendication 12, où l'anticorps est spécifique pour la protéine sFRP4.

14. Utilisation selon la revendication 10 ou 11, où l'anticorps est capable de se lier aux protéines sFRP1, sFRP2, sFRP3, sFRP4 et sFRP5.

15. Utilisation selon l'une quelconque des revendications 10 à 14, où l'anticorps est immobilisé sur une phase solide.

16. Utilisation selon la revendication 15, où la phase solide est une bande d'essai.

17. Utilisation selon l'une quelconque des revendications 10 à 16, où ledit kit comprend également un deuxième anticorps capable de se lier spécifiquement à la protéine sFRP4.

18. Utilisation selon l'une quelconque des revendications 10 à 17, où ledit kit comprend également un concentré de tampon de lavage et/ou une composition comprenant la protéine sFRP4.
